# EUROPEAN PATENT SPECIFICATION

(11) **EP 2 452 698 B1**
(45) Date of publication and mention of the grant of the patent: **21.02.2018**
(21) Application number: 11188682.6
(22) Date of filing: 10.11.2011
(51) Int. Cl.: A61L 26/00, A61K 38/42

(54) **Novel method for treatment of skin wounds and preparations for implementation of same**
Neuartiges Verfahren zur Behandlung von Hautwunden und Präparate zu ihrer Implementierung
Nouveau procédé pour le traitement des plaies et préparations relatives au procédé

(30) Priority: 12.11.2010 US 412993 P
(43) Date of publication of application: 16.05.2012
(73) Proprietor: Khorionyx, 69890 La Tour de Salvagny (FR)
(72) Inventor: Tayot, Jean-Louis, 69890 La Tour de Salvagny (FR)
(74) Representative: Lavoix

(56) References cited:
- WO-A1-87/05809
- US-A1- 2007 031 474
- US-A1- 2009 312 239

## Description

This invention relates to a novel use of a preparation in the treatment of skin wounds. This invention is intended to simplify the methods for treatment of skin wounds in order to improve the healing thereof, facilitate care and reduce costs of same.

Numerous healing products and numerous methods for treating skin wounds have already been described. They are generally characterized by the local application of dressings that must be renewed each day or a number of times per week, after having carefully cleaned the wound, so as to prevent local infection, which is detrimental to healing, and which can have formidable consequences for the patient. The use of active biological products on the healing mechanisms, in particular on the stimulation of the granulation tissue and epidermisation of the wound has led to significant progress. Among these products, platelets, growth factors, tissue extracts, platelet-derived growth factors (PDGF) or cell growth factors (EGF, FGF, etc.) have found numerous outlets in a vast market. However, these active molecules must be applied by means of a biological substrate or a matrix that is preferably bio-resorbable, in order to protect them and enable their progressive diffusion in the wound. Among these substrates, collagen, oxidized cellulose, alginates, chitosan or other polysaccharides, for example, have been described. It has also been proposed to use globin preparations insoluble at neutral pH, which, in addition to ensuring wound filling, facilitate tissue regeneration; see the application and patent WO 2004 100934 and US 6 949 625.

It is, however, known that biological substrates can activate microbial proliferation in contact with the wound and necessitate frequent changes and cleanings, which increase the consumption of products and the time and cost of care. Among these substrates, only chitosan is known for its bactericidal properties, which may make it preferable. However, this bactericidal effect is generated by a toxic action in contact with the bacterial membrane, which can also produce a toxic effect on the cell membranes. In addition, the resorption of chitosan in contact with the dermal tissue leads to a local inflammatory reaction that hinders the speed and quality of healing.

For all of these products, the addition of topical or general antibiotics is sometimes necessary, but not always desirable or sufficient, in order to fight infection of the wound. This is the case, for example, in large burns and for chronic wounds of poorly vascularised tissue. In addition, the frequent changing of dressings associated with washing of wounds by healthcare providers is very laborious and costly. Finally, they have another major disadvantage, which is the disruption of the granulation tissue or the neo-epidermis being formed.

The bactericidal and fungicidal properties of human haemoglobin and its peptide fragments have already been described, in particular in the following publications: Liepke C et al. (2003): Human hemoglobin-derived peptides exhibit antimicrobial activity: a class of host defence peptides. J Chromatogr B - Analyt Technol Biomed Life Sci., 791(1-2), 345-356. This publication characterizes haemoglobin as well as two peptides derived from haemoglobin as gram+ and gram- bacterial growth inhibitors, as well as *Candida albicans* yeast. These controls are performed by diffusion of soluble active principles in gels containing the bacteria. These authors conclude that haemoglobin and these peptide derivatives may be important elements in the immune response and innate, stopping microbial development.

Parish C.A. et al. (2001): Broad-Spectrum antimicrobial activity of haemoglobin, Bioorganic & Medicinal Chemistry, 9, 377-382. This publication can be summarized as follows:
Haemoglobin tetramers, in particular human, have an important activity against gram+ and gram- bacteria and fungal agents. In every case, the separate activity of the separate sub-units, alpha and beta chains of globin, is at least as strong as that of the intact haemoglobin tetramer. The fragments obtained by hydrolysis with cyanogen bromide still have a significant antimicrobial activity. The carboxy-terminal portion of thirty amino acids in α-helical cationic form is active against *Escherichia coli, Staphylococcus aureus* and *Candida albicans.* In conclusion, the fact that different peptide fragments of haemoglobin have diverse antibiotic properties tends to prove that the haemoglobin acts, in addition to its oxygen carrier functions, as a powerful agent in the defence against numerous microorganisms.

Hoffman et al. (2002), Mammalian-derived peptides for the treatment of microbial infections. US patent application 09/352078, filed on July 14, 1999. US Patent: 6337314, granted on Jan 8, 2002. The invention describes the antimicrobial properties of haemoglobin as well as of the alpha and beta chains, fragments obtained by hydrolysis of cyanogen bromide and corresponding synthetic peptides. These products are active against gram+ bacteria such as *Staphylococcus aureus* and *Streptococcus faecalis,* against gram- bacteria such as *Escherichia coli* and *Pseudomonas aeruginosa,* and against *Candida albicans* yeast. They are comparable in this way to the antimicrobial peptides secreted by neutrophilic leukocytes such as cathepsin D and azurocidin. The characterization of these properties is performed by diffusion of the soluble active product in an agar containing the microbial agents. The proposed mode of administration is in particular by intravascular injection in order to obtain a general effect.

However, the soluble character of haemoglobin or its peptide derivatives as described in the previous publications or patents cannot enable them to remain in the wound, due to very rapid tissue or blood diffusion. Their antimicrobial activity on the wound can be only brief and make them relatively or completely inactive in the long-term maintenance of the sterility of the wound.

US 2007/0031474 A1 describes a preparation which can be used for topical application to open wounds of the skin to stimulate healing of said wounds. The preparation comprises a mixture of globin which is soluble at physiological pH, and of insoluble globin. This preparation is used to fill wounds. The wounds are cleaned with physiological water every 3 to 4 days, but the globin preparation, is neither removed nor replaced and remains completely integrated in the wound. The invention proposes overcoming these disadvantages and providing a method for treatment of skin wounds, including deep wounds, making it possible to eliminate or reduce the interventions designed to clean the wounds, disinfect them and renew the substrates, while facilitating proper healing and tissue repair.

The invention relates to a preparation for use in the treatment of skin wounds as defined in claim 1, in which an effective amount of an insoluble globin preparation is placed in direct contact with the skin wound. The preparation coats and/or fills the wound. The preparation is kept in place for at least one week, a period of time during which it is not necessary to change or wash or clean the wound. The method allows to avoid a local infection that would be detrimental to the patient. This result, verified by means of a recognized animal model, is novel and surprising. The preparation may include other products as it will be disclosed hereinafter.

The invention thus relates to a preparation comprising insoluble globin for use in filling or coating a skin wound and exerting a local anti-infectious effect, which preparation is intended to be placed in direct contact with the skin wound. According to a feature, the preparation is kept in place for at least one week,. This preparation may be used as a drug or as a medical device.

Thus, during this period of time the preparation is kept in place, the preparation does not need to be changed or the wound cleaned.

Among the skin wounds that can be treated according to the invention, in particular, are diabetic ulcers or wounds, varicose ulcers or wounds, burn wounds and scars.

The insoluble globin is preferably insoluble natural globin which spontaneously precipitates at physiological or neutral pH, or at a pH close to neutral pH, in the range substantially between pH 5 and pH 8.

In the present invention, it is specified that the term "globin" is used in its scientific meaning which designates the whole proteic constituent of haemoglobin, once the heme separated. This term does not designate the sub-constituents such as the alpha or beta species or their fractions or mixtures prepared therefrom.

This globin obtained through elimination of heme from haemoglobin may be under two forms, say one major form insoluble at neutral or physiological pH, or at a pH in a range of pH of between about 5 and about 8, and one minor form that is soluble within this pH range. The insoluble globin is the component of interest for the preparation according to the invention. However, the presence of the minor soluble component is not detrimental. It can be however readily eliminated and thus not present in the preparation.

The insoluble character of the globin used in the invention prevents it from diffusing in an agar containing target bacteria. Therefore, the search for the bactericidal action of insoluble globin by this technique remained negative. It was not obvious that the phagocytosis of insoluble globin by a given population of bacteria would suffice to kill them. This differentiates the mode of action of insoluble globin with respect to soluble haemoglobin or separate globin chains, or peptide fragments of globin chains. The insoluble globin can manifest its bactericidal and fungicidal properties only at the site and after having been phagocytised, transformed and used by the infectious agent.

The use of human globin, alone or in combination with the existing products, makes it possible to preserve the dressing thus produced in place on the wound during a long period of time, in particular for one to four weeks, without requiring other nursing care. It is preferable for the preparation to remain in place for at least fifteen days, for example three or four weeks or even more. In an embodiment, the preparation is maintained for at least two weeks. In another embodiment, the preparation is maintained for at least one month. In another embodiment, the preparation is replaced one to four times a month. It makes it possible to save on corresponding costs and improve the quality and speed of healing. This method is based on the bactericidal and fungicidal properties of insoluble globin, without adverse effects on the cell membranes of the tissue. The insoluble character of the globin is necessary. The insoluble globin makes it possible to prevent its diffusion in the neighbouring tissue and preserve its properties in the wound. This constant presence of insoluble globin on or in the granulation tissue being formed makes it possible to prevent any local development of most gram+ or gram- bacteria or other pathogenic agents such as yeast.

The addition of haemoglobin or soluble globin is possible and can provide an advantage by enabling an additional antimicrobial action at a distance, which can improve the disinfection of tissue adjacent to the wound. However, this action is only brief. Haemoglobin is very easy to obtain since it can be purified before the preparation of the insoluble globin. Some of this haemoglobin solution can therefore be put on hold so as to be mixed in the end with the insoluble globin paste. The naturally soluble globin is very unstable but can be chemically modified in order to become stable, in particular by succinylation or esterification; see WO 2007 017580 and US 7 709 017.

Preferably, the haemoglobin and/or soluble globin content, in the preparation, will be less than or equal to 5% by weight, and especially preferably between 0.5% and 2% by weight. Such a preparation can be made in advance, but it can also be made at the time of intervention, for example by using the globin-based contents of a preparation syringe, for example in paste or powder form with a haemoglobin solution syringe.

The haemoglobin can come from blood bags or red cells pouches, but it can also very easily be autologous, by preserving some of the patient's haemoglobin that is produced during the production of insoluble globin with a sample of the patient's blood.

In the implementation of the invention, the globin can form the exclusive or main component of the preparation for filling the skin wound, or be combined with other materials or products for this role, for example collagen, oxidized cellulose, alginates, chitosan or other polysaccharides. The preparation may also comprise a pharmaceutically acceptable vehicle or excipient.

In such combinations with another material or product, it is preferable for the insoluble globin to form at least 50% by weight, but it is also possible to use lower contents, as the globin still makes it possible to supply the limits of these materials in order to facilitate cell regeneration or protection against bacteria and fungi.

For the implementation of the treatment method according to the invention, the preparation can be, for example, in the form of paste, gel, powder, granules, sponge, film, or coating. As an example, it is possible to use a paste, gel, powder or granules or sponges, covered either in production or by the practitioner, with a protective coating, for example a textile or a film. This coating can in particular be based on globin or chitosan.

The preparation may thus comprise from 5% to 100% by weight of insoluble globin.

In the case of a solid form, such as powder, granules, sponge, film or coating, it is preferred that the insoluble globin represents 50% by weight or more of the preparation, up to 100% by weight.

The preparation containing globin can also comprise active healing products or cells, for example, platelets, platelet-derived growth factors (PDGF) present in a platelet-rich plasma preparation or recombinant product, or cell growth factors (EGF, FGF, etc.).

Globin is the protein constituting haemoglobin, which itself contains four peptide chains (2 alpha and 2 beta chains) each associated with a haem. The haem consists of a tetrapyrrole structure containing 1 positively charged iron atom. There are 4 haems per molecule, responsible for the red colouring of haemoglobin. The methods for preparing globin have been known for a long time and have been developed for the purpose of dietary applications or for the preparation of injectable pharmaceutical solutions of chemically modified soluble globin.

Unlike haemoglobin, which is perfectly soluble at physiological pH, globin is mostly insoluble under the same conditions. Recently, new materials and new medical applications have been described based on globin that is insoluble at neutral pH under physiological conditions; see the application WO 2004 100934 and US patent 6 949 625. See also the application FR-A-2 854 801.

This invention requires the insoluble character, which is natural at a pH of between 5 and 8, of globin to be preserved, for example, by collecting, by centrifugation, a protein precipitate of globin formed by suspension of said precipitate in a pharmaceutically acceptable vehicle, preferably an aqueous vehicle, for example an aqueous physiological solution, containing 9 g/l of NaCl and buffered to a pH of between 5 and 7.5. The originality and benefit of this product lie in the fact that it is a protein concentrate, perfectly biocompatible with the surrounding tissue in or on which it is applied. This protein was not subject to any chemical alteration or modification, and is primarily insoluble once it is in a physiological environment.

Globin of animal origin, such as bovine or porcine, can be used but homologous human globin is preferable and enables any immunological reaction of the patient to be treated to be avoided. Globin may be autologous globin obtained from the patient's blood.

The globin however remains soluble at acid or basic pH and under these conditions can be filtered sterilely on porous membranes. For suitable concentrations of 20 to 300 mg/ml, such solutions can be treated as protein solutions and make it possible to produce products such as: sponges, films, coatings or granules by using or combining the drying, lyophilisation, cross-linking and precipitation techniques. The globin can be combined with other active principles or materials.

Human globin is easy to purify from human red cells. These are available in a sufficient quantity from red cell donations or expired whole blood, remaining in storage at blood transfusion centres and for which all preliminary sanitary tests were conducted at the time of sampling. The preparation of insoluble globin or other globin-based biomaterials therefore represents novel medical applications enabling expired blood donations to be beneficiated and the destruction thereof to be avoided or reduced.

The implementation of the invention is also possible from a blood sample of around 10 to 400 ml taken from the patient to be treated. Its transformation into autologous globin is performed with the same methods as for large volumes. For autologous blood, tests for detecting infectious agents can be simplified if traceability is guaranteed.

To implement the invention, the red cells from these blood samples can first be collected and purified by simple operations that are already known. The red cells are recovered by low-speed centrifugation. The plasma supernatant is separated and replaced by a PBS-type physiological saline liquid containing 9 g/l of NaCl and buffered at neutral pH. After a number of washings (3 to 5), the red cell suspension is thus freed of plasma proteins. One or two volumes of distilled water are added to the purified red cells pellet in order to produce an osmotic shock that leads to lysis of the red cell membranes and releases the haemoglobin in a concentrated and purified solution. A high-speed centrifugation step (5000 to 20,000 rpm) makes it possible to remove the membrane and cell debris in the centrifugate. A final step of filtration of the supernatant on a membrane with a porosity of 0.45 to 0.2 microns makes it possible to prepare a purified and sterile haemoglobin solution, free of particles and debris derived from tissues, cells or membranes.

The haemoglobin cleavage is performed, for example, by precipitation of the haemoglobin in at least 10 volumes of acid acetone according to one of the following examples. The globin thus prepared is soluble at acid or alkaline pH but becomes mostly insoluble once the pH of the aqueous solution is adjusted to between 5 and 8.

The insoluble character in a physiological medium explains the persistence of the globin after tissue implantation, which also makes it resistant to enzymatic degradation, in particular if a large amount is used, which is the case in skin healing applications.

The verification of the benefit of the invention can easily be performed using an animal globin preparation applied to deep skin wounds produced preferably on an animal of the same species. Several examples below show the good results obtained. The application of globin alone or in combination with conventional dressings makes it possible to reduce the number of interventions on the wound treated. The changing of the dressing and the superficial washing of the wound can be eliminated or reduced to a frequency of 1 to 4 times per month, without local infection.

The pH range of the globin preparations is from 5 to 8.

For preparations in the form of pastes, or gels, the content of insoluble globin is preferably from 5% to 15% by weight.

For chronic wounds that heal slowly and that are infected, it is preferable to use, in a first stage, a globin preparation at a pH of between 5 and 6, because its bactericidal power is broader in this pH range. The active healing of the wound is possible only in a second stage, when it is free of bacteria. In this second part, it is preferred to use a globin preparation at neutral physiological pH (6.5 to 7.5) which makes it possible to prevent any bacterial or fungal development and the wound can heal quickly, without any subsequent changing of the dressing.

Thus the invention relates to a preparation for use in the treatment of skin wounds, especially a chronic skin wound, wherein an effective amount of an insoluble globin preparation is placed in direct contact with the skin wound, the preparation is kept in place for at least one week, wherein the pH of the preparation is in the range of 5 to 6, this preparation is then removed and then an effective amount of an insoluble globin preparation is placed in direct contact with the skin wound, wherein the pH of the preparation is in a range from 6.5 to 7.5.

The invention also relates to a preparation comprising insoluble globin for use in filling or coating a skin wound, especially a chronic skin wound, wherein the preparation is used in two times, with a first preparation having a pH between 5 and 6 for at least one week, then a second preparation having a pH between 6.5 and 7.5 for at least one week.

### Examples of embodiments of the invention

### Example 1: Preparation of a human globin paste, insoluble at physiological pH

10 mL of human blood are collected by venous puncture of a patient and collected under agitation in 4 tubes each containing 0.3 mL of sodium citrate or EDTA powder. This sampling with anticoagulant can be preserved for at least two weeks at +4°C before use. Four volumes of a sterile physiological solution are added to the blood, then it is centrifuged for 3 minutes at 2000 rpm. The plasma protein supernatant is removed. The washing operation is repeated three times to remove the plasma proteins. The purified cell pellet is then haemolysed by adding a volume of sterile distilled water. The haemoglobin solution is then centrifuged for 30 minutes at 5000 rpm to remove cell and membrane debris, then the supernatant is clarified by filtration on a pre-filter and filtered sterilely on a membrane with a porosity of between 0.2 and 0.45 microns. After rinsing of the filter with sterile water, a volume of 38 ml is obtained, which can be preserved at +4°C. This solution is slowly poured under agitation into 400 mL of acetone, containing 4 mL of HCI 12 N. The suspension is agitated vigorously and left to rest for 10 minutes at room temperature, under a chemical hood or in a sealed reactor. The acetone containing the dissolved haem is removed by filtration on a porous cloth. The faded globin precipitate is washed on the cloth by anhydrous acetone and recovered after acetone removal. A vacuum drying can make it possible to prepare the white globin powder, which is easy to store or transport, if necessary. The globin acid precipitate, or the corresponding acid powder, is then re-dissolved in 35 mL of sterile distilled water. The solution is then adjusted to pH 7.0 by adding 1N NaOH and the globin precipitates massively. Optionally, an alkaline treatment with a final concentration equal to 0.2 N NaOH, for 1 hour at +20°C, followed by precipitation at neutral pH by adding hydrochloric acid 1N, makes it possible to ensure the additional inactivation of infectious or transmissible agents that may be present in the patient's blood. The purified neutral globin precipitate is washed on a filtration cloth by a physiological solution of NaCl 9g/l solution. The concentration of the paste can be adjusted by drying and weighing the end product. Finally, the insoluble globin is in suspension in a physiological solution of sodium chloride 9 g/l, optionally buffered to ph 7. The paste obtained weighs around 10 grams and occupies a volume of 10 mL. Its usual concentration is around 8%. By extending the drying in the final filtration, the concentration of the paste can be increased to 10% or even 12% in order to give an adapted consistency to the application on different types of skin wounds that are more or less deep, and for example, prevent the overflow of paste on shallow and more or less inclined wounds. Finally, the insoluble globin paste can be sterilized by beta or gamma irradiation at a dose of 5 to 30 kGray, if the conditions for preparation thereof do not ensure sterility.

### Example 2: Preparation of a human globin paste, insoluble at a pH of between 5 and 6

Example 1 is repeated with the following variation at the time of dissolution of the globin powder or acid precipitate. The globin acid precipitate or the corresponding acid powder is re-dissolved in 35 mL of sterile distilled water. The solution is then adjusted to a pH between 5 and 6 by adding 1N NaOH and the globin precipitates massively. The globin paste at pH between 5 and 6 is prepared according to the same conditions as in example 1.

### Example 3: Demonstration of bactericidal and fungicidal properties of human globin insoluble at physiological pH

Microbiological tests on the insoluble globin paste of example 1 were conducted as follows. The globin paste is sterilely distributed in 1-ml Becton Dickinson Luer Lok Tip syringes. *Staphylococcus aureus* and *Candida albicans* suspensions in precise concentrations are prepared, controlled and distributed in other 1-mL B.D. syringes. 0.1 mL of each microbial suspension are mixed with 0.9 mL of globin paste in order to have an inoculum of between 10 and 500 microorganisms per globin syringe. The mixture is produced by a sterile double Luer connector (ref: FTLLC-6 Promepla, Principality of Monaco) joining the two syringes and a dozen homogenization cycles from one to the other syringe. The mixtures are incubated for 7 days at 37°C. After 7 days, samples from the contaminated globin syringes are seeded on Petri dishes containing a TSA medium for the search for *Staphylococci* or an SDA medium for the search for yeast. The microbial control suspensions are seeded in the same way in order to verify their concentration. No colony developed after 48 hours for the seedings containing globin. These results confirmed by a plurality of experiments demonstrate the inactivation of *Staphylococci* (Gram+) and *Candida albicans* yeast by neutral globin after 7 days of incubation at 37°C.

### Example 4: Demonstration of the bactericidal and fungicidal properties of human globin insoluble at pH 5.5

The microbiological tests on the insoluble globin paste of example 2 were conducted as follows. The globin paste is sterilely distributed in 1-ml Becton Dickinson Luer Lok Tip syringes. *Staphylococcus aureus, Pseudomonas aeruginosa* and *Candida albicans* suspensions in precise concentrations are prepared, controlled and distributed in other 1-mL B.D. syringes. 0.1 mL of each microbial suspension are mixed with 0.9 mL of globin paste at pH 5.5 in order to have an inoculum of between 10 and 500 microorganisms per globin syringe. The mixture is produced by a sterile double Luer connector (ref: FTLLC-6 Promepla, Principality of Monaco) joining the two syringes and a dozen homogenization cycles from one to the other syringe. The mixtures are incubated for 7 days at pH 5.5 and at 37°C. After 7 days, samples from the contaminated globin syringes are seeded on Petri dishes containing a TSA medium for the search for *Staphylococci* and *Pseudomonas* or an SDA medium for the search for yeast. The microbial control suspensions are seeded in the same way in order to verify their concentration. No colony developed after 48 hours for the seedings containing globin. These results confirmed by a plurality of experiments demonstrate the inactivation of *Staphylococci* (Gram+), *Pseudomonas* (Gram-) and *Candida albicans* yeast by globin after 7 days of incubation at pH 5.5 and at 37°C.

A different control method was also tested on the same globin paste. Decreasing concentrations of luminescent bacteria (*Staphylococcus aureus* Xen29, supplied by Caliper Life Sciences) are added to the paste in six 1-mL syringes. In parallel, the same amounts of *Staphylococcus Aureus* Xen29 are added to syringes of Tryptone Soy culture medium and collagen. The concentrations tested vary from 10¹⁰ to 10⁵ bacteria/mL and two series of tests are conducted in parallel at 37°C for each of the products. At different times: T0, T0+15 min, T0+30 min, T0+40 min, T0+1 h, T0+2 h, T0+4 h and T0+6 h, the flow of photons emitted by a precise surface (4.2 x 0.94 mm²) of each syringe is recorded. The results show that the globin paste is the only product of the series that enables the photon flow to be stopped below the sensitivity threshold of the method, for the highest concentrations tested. This suppression of the photon flow is due to a bactericidal effect of the globin and not to a simple inactivation that may come from the lack of growth medium.

In order to check the *in vivo* bactericidal and fungicidal properties, it is possible at the end of the experiment to inject in suitable amounts the contents of the globin syringes subcutaneously into mice and then to check the lack of restoration of the photon flux. In parallel, it is also possible to prepare syringes of the globin paste contaminated by 10⁸ *Staphylococcus* Xen29 bacteria, to inject in suitable amounts and immediately after the contamination the content of these syringes in other mice and to check whether the bioluminescence (visible at the beginning below the skin of these mice) disappears, for example in two hours.

### Example 5: Preparation of human globin powder, insoluble at pH between 5 and 7.5

The method of example 1 is used until filtration of the globin acetone precipitate. The faded human globin precipitate is washed on the cloth by anhydrous acetone and recovered after acetone removal. The globin acid precipitate is then re-dissolved in 10 mL of sterile distilled water. The solution is then adjusted to a pH between 5 and 7.5 by adding 1N NaOH and the globin precipitates massively. The globin suspension can be concentrated and thickened by washing with distilled water and sedimentation. It is then lyophilised. Finally, the powder obtained can be sterilized by beta or gamma irradiation at a dose of 5 to 30 kGray, if the conditions for its preparation do not ensure its sterility. The purified globin powder weighs around 1 gram and can preserve a certain cohesion if the concentration of the suspension to be lyophilised is sufficient. Before lyophilisation, the superimposition of the globin suspension by a polysaccharide solution or suspension, such as oxidized cellulose, sodium alginate or chitosan makes it possible to obtain a two-layer compress. The face formed by globin must be applied in direct contact with the wound, with the upper polysaccharide layer forming an external protection. It is all covered with a liquidproof film, permeable to air, in order to protect the dressing from external irritants or contamination.

### Example 6: Preparation of porcine globin powder, insoluble at pH between 5 and 7.5

The start of the method of example 1 is performed using porcine blood until filtration of the globin acetone precipitate. The faded globin precipitate is washed on a porous cloth by anhydrous acetone, and recovered after removal of the acetone. The porcine globin acid precipitate is re-dissolved in 30 mL of sterile distilled water. The solution is then adjusted to a pH between 5 and 7.5, by adding 1N NaOH and the porcine globin precipitates massively. The globin suspension is then homogenized, and then 10 volumes of acetone are progressively added. The globin precipitates, and the acetone precipitate is harvested by filtration on porous cloth, then washed with acetone and dried under a sterile laminar flow. The purified globin powder weighs around 1 gram. It can be finely divided with a spatula and then distributed in a powdering device or in 5-ml syringes with a Luer lock end piece, in an amount of 500 mg per syringe. Finally, this powder can be sterilized by beta or gamma irradiation at a dose of 5 to 30 kGray, if the conditions for its preparation do not ensure sterility. It can be applied to the pig's wound by sprinkling with a powdering device. The powder is then covered with a compress, preferably bio-resorbable, and a film providing protection against external irritants and contamination.

### Example 7: Preparation of a porcine globin paste, insoluble at pH 5.5, and application on porcine wounds

Example 6 above is performed, and the porcine globin powder is distributed in 5-mL syringes each containing 500 mg. Each syringe can then be connected to another 5-mL syringe containing 2 to 2.5 mL of a saline solution containing 9 g/L of NaCl, sterile, by means of a sterile double Luer connector (ref: FTLLC-6 Promepla, Principality of Monaco). After a dozen homogenization cycles between the two syringes, a porcine globin paste is obtained with a concentration of 8 to 12% and pH 5.5, which can be applied directly on skin wounds, with 2-cm sides, produced by complete ablation of the dermis, on a pig. The paste is then covered with a Tegaderm® film adhering to the healthy peripheral skin, which provides protection against external irritants and contamination. A Vetrap® bandage is applied around the animal's body to make sure that it stays in place on the animal. Each week, the external dressing is changed without touching, adding or washing the globin paste or the newly formed tissue. After 28 days, without any other handling, the healing and epidermisation of the wound are complete. None of the wounds thus treated develops a foul-smelling odour or infection visible when the dressing is opened.

### Example 8: Combination of a sterile porcine globin paste with a chitosan film

Example 7 above is reproduced by replacing the Tegaderm® film with a chitosan film. A Vetrap®-type bandage is applied above it to make sure that it stays in place on the animal. The dressing is left in place for 28 days, without any other manipulation, until healing and epidermisation of the wound. None of the wounds thus treated develops a foul-smelling odour or infection visible when the dressing is opened.

### Example 9: Combination of an insoluble globin paste with oxidized cellulose fibres

The porcine globin paste is prepared according to example 6. It is mixed with an oxidized cellulose fibre paste, in equal proportions by volume. It is all lyophilized and sterilized by gamma irradiation at a dose of 25 to 30 kGray. A mixed sponge of oxidized cellulose and insoluble globin is obtained for the treatment of skin wounds. The globin and oxidized cellulose can be modified in order to optimize the healing properties and the speed of resorption of the biomaterial.

### Example 10: Preparation of an insoluble human globin paste containing soluble human haemoglobin

The method of example 1 or 2, in part, is repeated using human blood. When the haemoglobin solution is purified and sterilized by filtration, a 5-ml sample is adjusted at a concentration of between 1 and 5% by weight and to a pH of between 5 and 7.5, then distributed in two sterile 5-mL syringes with a Luer Lock end piece, and the two syringes, each containing 2.5 mL, are put on hold. The preparation of the globin is continued on the rest of the haemoglobin solution, until filtration of the globin acetone precipitate. The faded human globin precipitate is washed on the cloth with anhydrous acetone, and recovered after drying. The globin acid precipitate is then re-dissolved in 30 mL of sterile distilled water. The solution is then adjusted to a pH between 5 and 7.5 by adding 1N NaOH and the globin precipitates massively. The globin suspension is then homogenized, then 10 volumes of acetone are progressively added. The globin precipitates and the acetone precipitate is harvested by filtration on a porous cloth then washed with the acetone and dried under a sterile laminar flow. The globin powder, purified, weighs around 1 gram. It can be finely divided with a spatula, and then distributed in two 5-ml syringes with a Luer lock end piece, in an amount of 500 mg per syringe. Finally, this powder can be sterilized by beta or gamma irradiation at a dose of 5 to 30 kGray, if the conditions for its preparation do not ensure sterility. Each globin powder syringe is then mixed with each haemoglobin syringe. This mixture is performed by means of a sterile double Luer connector (ref: FTLLC-6 Promepla, Principality of Monaco). After joining a dozen homogenization cycles between the two syringes, an insoluble human globin paste at a concentration of 8% is obtained, containing the soluble human haemoglobin. This preparation can be applied directly on skin wounds, according to the method already described. The preparation can be left in contact with the wound for at least one week, under its protective film, without any other intervention.

### Example 11: Preparation of an insoluble human globin paste containing chemically modified soluble globin

The previous example is performed by replacing the haemoglobin with a succinylated globin solution at neutral pH, prepared according to example 4 of the patent FR 05 0839∂2 or WO 2007 017580 or US 7 709 017.

Briefly, the insoluble human globin powder of the previous example is used at a concentration of 30 mg/ml, in a NaOH solution 1g/l containing 9 g/l of NaCl, then 80 mg of succinic anhydride is added per gram of globin, at a temperature of 20 °C. The pH decreases spontaneously to 9, where it is maintained by progressively adding NaOH, with continuous monitoring of the pH meter. The reaction is terminated when the pH is stable. After 1 hour of waiting, the solution is adjusted to a neutral pH and precipitated by an aqueous acetone solution at 90%. The precipitate is washed with anhydrous acetone and then dried. A neutral succinyl-globin powder is obtained. It is solubilized in a physiological solution at neutral pH. The clear solution obtained is sterilely filtered on a membrane with a porosity of 0.2 µ and then distributed in 5-mL syringes each containing 2.5 mL. The mixture with a new neutral insoluble globin powder can then be produced for application of the mixture on the wound to be treated.

### Example 12: Method for healing a skin ulcer with an insoluble human globin paste containing a hyaluronate gel

An insoluble human globin paste at 12% is prepared according to example 1, 2 or 5. It is then mixed with a sterile hyaluronate gel at 1%, for example in the proportion of 1 volume of hyaluronate for 9 volumes of globin. The hyaluronate with a molecular weight above 2000 k Dalton can be supplied by HTL, La Boitardière 35133 - Javene - France, or any other manufacturer. The hyaluronate is sterilely distributed in a sterile 1-ml Schott syringe with a Luer Lock end piece. The globin paste is preferably at pH 5.5 for the first portion of the treatment, in order to optimally disinfect the wound. The globin is in a 5-mL syringe with a Luer Lock end piece. The mixture is produced by means of a double Luer connector (ref: FTLLC-6 Promepla, Principality of Monaco). After a dozen homogenization cycles between the two syringes, the sterile mixture of the two biopolymers is applied directly on the ulcer to be treated. The paste is then covered with a Tegaderm® film adhering to the healthy peripheral skin, and a bandage providing protection from external irritants and contamination. The dressing is left in place for two weeks, without any other manipulation. Twice per month, the dressing is washed with the usual precautions. The wound is washed superficially, respecting the underlying tissue and leaving the traces of product still present. For the second part of the treatment, the wound is filled if necessary by a globin paste at physiological pH and hyaluronate, then covered by a new Tegaderm® film and a bandage, until healing and epidermisation of the wound. The wound thus treated does not develop a foul-smelling odour or infection visible when the dressing is opened.

## Claims

1. Insoluble globin preparation comprising natural globin that is insoluble at a pH from 5 to 8, for use in the treatment of skin wounds, namely chronic skin wounds or burn wounds and scars, by filling or coating said wound and exerting a local anti-infectious effect to avoid infection thereof, which preparation is placed in direct contact with said wound and kept in place for at least one week, without changing the preparation and without cleaning the wound.

2. Preparation for use according to claim 1, wherein the preparation comprises from 5 to 100 % by weight of insoluble globin.

3. Preparation for use according to claim 1 or 2, wherein the preparation is kept in place for at least two weeks.

4. Preparation for use according to claim 1 or 2, wherein the preparation is kept in place for at least one month.

5. Preparation for use according to any of claims 1 to 4, wherein the skin wound is chosen from the group formed by diabetic ulcers or wounds, varicose ulcers or wounds and burn scars or wounds.

6. Preparation for use according to any of claims 1 to 5, **characterized in that** the globin is a natural globin being insoluble at a pH range from 5 to 8.

7. Preparation for use according to any of claims 1 to 6, **characterized in that** the globin is a natural human globin.

8. Preparation for use according to any of claims 1 to 7, **characterized in that** the globin preparation is under paste or gel form.

9. Preparation for use according to claim 8, **characterized in that** the concentration of globin is in the range of 5% to 15% by weight.

10. Preparation for use according to any of claims 1 to 7, **characterized in that** the preparation is in the form of powder, granulates, coating, sponge or film.

11. Preparation for use according to any of claims 1 to 9, **characterized in that** the pH of the preparation is in the range of 5 to 6.

12. Preparation for use according to any of claims 1 to 9, **characterized in that** the pH of the preparation is in the range of 6.5 to 7.

13. Preparation for use according to any of claims 1 to 12, **characterized in that** the preparation also contains haemoglobin and/or globin being soluble at physiological pH, at a concentration of haemoglobin and/or soluble globin under 5% by weight of the preparation.

14. Preparation for use according to any of claims 1 to 13, **characterized in that** the preparation further comprises active wound healing products or cells, and/or wound filling materials.

15. Preparation for use according to any one of claims 1 to 9 or 11-14, wherein the preparation is intended to be used in two times, with a first preparation having a pH between 5 and 6 for at least one week, then a second preparation having a pH between 6.5 and 7.5 for at least one week.

## Patentansprüche

1. Präparat aus unlöslichem Globin, umfassend natürliches Globin, das bei einem pH-Wert von 5 bis 8 unlöslich ist, zur Verwendung bei der Behandlung von Hautwunden, nämlich chronischen Hautwunden oder Verbrennungswunden und -narben, durch Füllen oder Beschichten der Wunde und Ausüben einer lokalen antiinfektiösen Wirkung zum Verhindern einer Infektion derselben, wobei das Präparat in direkten Kontakt mit der Wunde platziert wird und mindestens eine Woche lang in Position belassen wird, ohne das Präparat zu wechseln und ohne die Wunde zu reinigen.

2. Präparat zur Verwendung nach Anspruch 1, wobei das Präparat von 5 bis 100 Gewichts-% unlösliches Globin umfasst.

3. Präparat zur Verwendung nach Anspruch 1 oder 2, wobei das Präparat mindestens zwei Wochen lang in Position belassen wird.

4. Präparat zur Verwendung nach Anspruch 1 oder 2, wobei das Präparat mindestens einen Monat lang in Position belassen wird.

5. Präparat zur Verwendung nach einem der Ansprüche 1 bis 4, wobei die Hautwunde aus der Gruppe gewählt ist, die durch diabetische Geschwüre oder Wunden, varikose Geschwüre oder Wunden und Verbrennungsnarben oder -wunden gebildet ist.

6. Präparat zur Verwendung nach einem der Ansprüche 1 bis 5, **dadurch gekennzeichnet, dass** das Globin ein natürliches Globin ist, das in einem pH-Bereich von 5 bis 8 unlöslich ist.

7. Präparat zur Verwendung nach einem der Ansprüche 1 bis 6, **dadurch gekennzeichnet, dass** das Globin ein natürliches menschliches Globin ist.

8. Präparat zur Verwendung nach einem der Ansprüche 1 bis 7, **dadurch gekennzeichnet, dass** das Globinpräparat in Form einer Paste oder eines Gels vorliegt.

9. Präparat zur Verwendung nach Anspruch 8, **dadurch gekennzeichnet, dass** die Konzentration von Globin in dem Bereich von 5 Gewichts-% bis 15 Gewichts-% liegt.

10. Präparat zur Verwendung nach einem der Ansprüche 1 bis 7, **dadurch gekennzeichnet, dass** das Präparat in der Form von Pulver, Granulat, Beschichtung, Schwamm oder Film vorliegt.

11. Präparat zur Verwendung nach einem der Ansprüche 1 bis 9, **dadurch gekennzeichnet, dass** der pH-Wert des Präparats in dem Bereich von 5 bis 6 liegt.

12. Präparat zur Verwendung nach einem der Ansprüche 1 bis 9, **dadurch gekennzeichnet, dass** der pH-Wert des Präparats in dem Bereich von 6,5 bis 7 liegt.

13. Präparat zur Verwendung nach einem der Ansprüche 1 bis 12, **dadurch gekennzeichnet, dass** das Präparat zudem Hämoglobin und/oder Globin, das bei physiologischem pH-Wert löslich ist, mit einer Konzentration von Hämoglobin und/oder löslichem Globin unter 5 Gewichts-% des Präparats enthält.

14. Präparat zur Verwendung nach einem der Ansprüche 1 bis 13, **dadurch gekennzeichnet, dass** das Präparat ferner Produkte oder Zellen zur aktiven Wundheilung und/oder Wundfüllmaterialien umfasst.

15. Präparat zur Verwendung nach einem der Ansprüche 1 bis 9 oder 11-14, wobei vorgesehen ist, dass das Präparat zwei Mal verwendet wird, wobei ein erstes Präparat, das einen pH-Wert zwischen 5 und 6 aufweist, mindestens eine Woche und dann ein zweites Präparat, das einen pH-Wert zwischen 6,5 und 7,5 aufweist, mindestens eine Woche verwendet wird.

## Revendications

1. Préparation de globine insoluble comprenant de la globine naturelle qui est insoluble à un pH de 5 à 8,
pour une utilisation dans le traitement de lésions cutanées, à savoir les lésions cutanées chroniques ou les lésions et les cicatrices par brûlure, par remplissage ou revêtement de ladite lésion et mise en oeuvre d'un effet anti-infectieux local afin d'éviter une infection de celle-ci, laquelle préparation est placée en contact direct avec ladite lésion et maintenue en place pendant au moins une semaine, sans changer la préparation et sans nettoyage de la lésion.

2. Préparation pour une utilisation selon la revendication 1, dans laquelle la préparation comprend de 5 à 100 % en poids de globine insoluble.

3. Préparation pour une utilisation selon la revendication 1 ou 2, dans laquelle la préparation est maintenue en place pendant au moins deux semaines.

4. Préparation pour une utilisation selon la revendication 1 ou 2, dans laquelle la préparation est maintenue en place pendant au moins un mois.

5. Préparation pour une utilisation selon l'une quelconque des revendications 1 à 4, dans laquelle la lésion cutanée est choisie dans le groupe formé par les ulcères ou les lésions diabétiques, les ulcères ou les lésions variqueuses et les cicatrices ou les lésions par brûlure.

6. Préparation pour une utilisation selon l'une quelconque des revendications 1 à 5, **caractérisée en ce que** la globine est une globine naturelle qui est insoluble dans une plage de pH de 5 à 8.

7. Préparation pour une utilisation selon l'une quelconque des revendications 1 à 6, **caractérisée en ce que** la globine est une globine humaine naturelle.

8. Préparation pour une utilisation selon l'une quelconque des revendications 1 à 7, **caractérisée en ce que** la préparation de globine est sous la forme d'une pâte ou d'un gel.

9. Préparation pour une utilisation selon la revendication 8, **caractérisée en ce que** la concentration en globine est comprise dans la plage allant de 5 % à 15 % en poids.

10. Préparation pour une utilisation selon l'une quelconque des revendications 1 à 7, **caractérisée en ce que** la préparation est sous la forme d'une poudre, de granulés, d'un revêtement, d'une éponge ou d'un film.

11. Préparation pour une utilisation selon l'une quelconque des revendications 1 à 9, **caractérisée en ce que** le pH de la préparation est compris dans la plage allant de 5 à 6.

12. Préparation pour une utilisation selon l'une quelconque des revendications 1 à 9, **caractérisée en ce que** le pH de la préparation est compris dans la plage allant de 6,5 à 7.

13. Préparation pour une utilisation selon l'une quelconque des revendications 1 à 12, **caractérisée en ce que** la préparation comprend également de l'hémoglobine et/ou de la globine qui est soluble au pH physiologique, à une concentration en hémoglobine et/ou globine soluble inférieure à 5 % en poids de la préparation.

14. Préparation pour une utilisation selon l'une quelconque des revendications 1 à 13, **caractérisée en ce que** la préparation comprend en outre des produits actifs ou des cellules permettant la cicatrisation d'une lésion et/ou des matériaux de remplissage d'une lésion.

15. Préparation pour une utilisation selon l'une quelconque des revendications 1 à 9 ou 11 à 14, dans laquelle la préparation est destinée à être utilisée en deux fois, avec une première préparation ayant un pH compris entre 5 et 6 pendant au moins une semaine, puis une seconde préparation ayant un pH compris entre 6,5 et 7,5 pendant au moins une semaine.
